# FASCICULE DE BREVET EUROPEEN

(11) **EP 0 998 314 B1**
(45) Date de publication et mention de la délivrance du brevet: **06.03.2002**
(21) Numéro de dépôt: 98940317.5
(22) Date de dépôt: 22.07.1998
(51) Int. Cl.: A61L 31/00

(54) **PROTHESE COMPOSITE POUR LA PREVENTION DES ADHERENCES POST-CHIRURGICALES ET SON PROCEDE D'OBTENTION**
KOMPOSIT-PROTHESE ZUR VERHINDERUNG POSTCHIRURGISCHER VERKLEBUNG UND VERFAHREN ZU IHRER HERSTELLUNG
COMPOSITE PROSTHESIS FOR PREVENTING POST-SURGICAL ADHESIONS AND METHOD FOR OBTAINING SAME

(30) Priorité: 01.08.1997 FR 9710104
(43) Date de publication de la demande: 10.05.2000
(73) Titulaire: SOFRADIM PRODUCTION, 01600 Trévoux (FR); Imedex Biomateriaux, 69630 Chaponost (FR)
(72) Inventeur: ORY, François, Régis, F-69270 Fontaines Saint Martin (FR); THERIN, Michel, F-69002 Lyon (FR); GRAVAGNA, Philippe, F-69450 Irigny (FR); TAYOT, Jean-Louis, F-69890 La Tour de Salvagny (FR)
(74) Mandataire: Guerre, Dominique
(86) Numéro de dépôt international: FR9801626
(87) Numéro de publication internationale: WO9906080

(56) Documents cités:
- EP-A- 0 372 969
- WO-A-89/08467
- WO-A-95/18638
- WO-A-96/08277

## Description

La présente invention concerne une prothèse composite pour la prévention des adhérences post-chirurgicales, et trouve application notamment dans le domaine de la chirurgie viscérale ou pariétale. L'invention sera décrite à titre d'exemple par rapport à une prothèse composite destinée à une utilisation en chirurgie pariétale, dans la réparation des éventrations, ou hernies.

Les adhérences post-chirurgicales comprennent toutes les liaisons fibreuses non anatomiques, induites fortuitement par un acte chirurgical lors du processus normal de cicatrisation. Elles peuvent survenir dans toutes les disciplines chirurgicales quel que soit le geste considéré. Elles sont généralement d'autant plus sévères que le traumatisme chirurgical est important et que les tissus assurant normalement les plans de clivage (tissu conjonctif interstitiel, les synoviales, les gaines tendineuses, séreuses péritonéale et pleurale...) ont été touchés. Tout traumatisme chirurgical tissulaire est suivi d'une cascade d'évènements physiologiques dont les principaux temps peuvent être simplifiés comme suit :
- temps zéro (t0) : traumatisme chirurgical, effraction capillaire ;
- temps zéro plus quelques minutes : coagulation, formation du réseau fibrineux, libération des facteurs chimiotactiques ;
- temps zéro (t0) plus 12 à 48 heures : afflux leucocytaire à dominante polynucléaire ;
- temps zéro (t0) plus 24 heures à 5 jours : afflux leucocytaire à dominante macrophagique ;
- temps zéro (t0) plus 4 à 8 jours : afflux fibroblastique ;
- temps zéro (t0) plus 5 à 14 jours : différenciation conjonctive de la réaction cicatricielle ;
- temps zéro (t0) plus 15 à 180 jours : remodelage cicatriciel.

Même si les mécanismes exacts sont pour certains encore inconnus, notamment en ce qui concerne le déterminisme de l'intensité de la réaction, il apparaît donc que les premiers jours sont déterminants puisqu'ils conditionnent l'afflux fibroblastique responsable de la formation d'adhérences.

De ce fait, de telles adhérences post-chirurgicales peuvent provoquer des syndromes pouvant se classer principalement en douleurs chroniques, syndromes occlusifs, et infertilité féminine. Par ailleurs, elles augmentent très sensiblement les risques de fausses routes lors d'une réintervention, (effraction myocardique ou intestinale lors de la thoracotomie ou laparotomie itérative), tout en prolongeant les temps opératoires, la dissection préalable pouvant être dans de tels cas très fastidieuse.

Une solution à ce problème consiste en l'interposition d'une barrière physique entre les structures que l'on souhaite ne pas voir adhérer. L'effet barrière recherché pose toutefois le problème du pouvoir adhésiogène intrinsèque de cette barrière. En effet, si la barrière est constituée d'un matériau non résorbable, elle peut être à l'origine elle même d'adhérences au cours du temps; et si elle est résorbable, sa résorption doit être suffisamment peu inflammatoire pour ne pas engendrer elle-même des adhérences.

Plusieurs propriétés sont donc nécessaires pour qu'un matériau puisse prétendre réduire le risque d'adhérences, à savoir, entre autres :
- le matériau dont il est constitué ou composé doit être sensiblement lisse et non poreux sur au moins l'une de ses faces, de façon à ne pas offrir d'espace à une recolonisation cellulaire ;
- la surface du matériau doit limiter l'adhésion cellulaire primitive.

Afin de remédier à ce problème, on a eu recours à des polymères artificiels hydrophobes et inertes, par exemple en PTFE expansé, ou à des substances polymères résorbables, par exemple à base de hyaluronates, ou de cellulose modifiée, qui forment rapidement un hydrogel par hydratation dans le corps.

Toutefois, et notamment dans la chirurgie viscérale et pariétale, mais aussi dans la chirurgie orthopédique ou neurologique, la barrière doit également présenter une certaine résistance mécanique lui permettant de remplir sa fonction en tant qu'élément de reconstruction chirurgicale. De manière générale, les tissus prothétiques connus notamment dans le traitement des insuffisances pariétales, par exemple hernies et éventrations, apportent un complément de résistance mécanique à la reconstruction chirurgicale. De tels tissus sont d'autant plus efficaces et leur tolérance locale d'autant meilleure que leur intégration tissulaire est intime et précoce. Pour cette raison les tissus prothétiques connus les plus performants dans ces indications sont généralement très poreux, et conçus de façon à être intégrés dans le corps le plus rapidement possible. Par "poreux", on entend la caractéristique selon laquelle au moins l'une des faces du tissu est rugueuse, pour présenter des alvéoles, réparties régulièrement ou non, et favorisant toute colonisation cellulaire. C'est ainsi que lors du contact avec les viscères par exemple, ces tissus sont adhésiogènes, ce qui limite leur utilisation aux sites dits pré- ou rétropéritonéaux. Or, dans un certain nombre de cas, et plus particulièrement lors d'éventrations multirécidivées, l'implantation en site prépéritonéal strict est difficile, voire impossible du fait de l'existence d'un déficit en séreuse étendu.

Un besoin s'est donc fait ressentir de disposer d'un produit permettant de résoudre le problème de prévention des adhérences post-chirurgicales, tout en offrant un renfort prothétique soumis à la recolonisation cellulaire et intégration tissulaire, et pouvant servir, par exemple, à traiter une éventration avec perte péritonéale importante, ou encore des petites éventrations par laparoscopie, et des hernies.

La demande de brevet WO-A-96/08277 décrit à cette fin une prothèse composite comprenant un tissu prothétique, en l'occurrence un treillis résorbable ou non, et au moins un film d'un matériau collagénique réticulé, en l'occurrence un gel de collagène coagulé à l'état sec, associé à une face du tissu prothétique. La prothèse composite ainsi constituée trouve application dans le traitement des éventrations et des hernies, et évite, selon les inventeurs, des adhérences postopératoires, car la membrane collagénique constitue une zone de clivage permettant la libération des éventuelles adhérences post-opératoires précoces qui peuvent se former. Il ressort des expériences menées sur des porcs, et décrites dans la demande, que le temps de résorption de la membrane collagénique demeure important.

Toute résorption de matériel étant plus ou moins pro-inflammatoire, la persistance au delà d'une dizaine de jours d'un matériau résorbable peut induire un retard de disparition des cellules inflammatoires sur le site. Cette présence maintenue de cellules inflammatoires (essentiellement macrophagiques) actives va déclencher une cascade d'activation conduisant à la stimulation des fibroblastes, eux-mêmes responsables de la formation directe des adhérences. Alors que les cellules mésothéliales, qui sont responsables du recouvrement péritonéal, sont connues pour leur faculté de régénération rapide (apparition dès le cinquième ou sixième jour), il est donc non seulement inutile, mais aussi sensiblement contraire aux principes de la prévention des adhérences de maintenir le matériau sur lequel repose l'effet barrière au-delà des huit à dix jours post-opératoires.

Ainsi, l'utilisation de la membrane collagénique associée au treillis synthétique telle que décrite dans la demande de brevet précitée peut être dans certains cas responsable d'adhérences initiales du fait de sa résorption relativement lente, et peut alors ne pas convenir pour résoudre le problème de prévention des adhérences post-chirurgicales, tout en offrant un renfort prothétique soumis à la recolonisation cellulaire et intégration tissulaire. Par ailleurs, sa résorption relativement lente limite une recolonisation efficace et précoce du treillis synthétique, celui-ci étant masqué par la membrane collagénique au moment où il doit être intégré, c'est-à-dire dans les deux premières semaines.

Par conséquent l'invention a pour objet une prothèse composite du genre défini dans le document WO-A-96/08277, permettant une intégration tissulaire effective, demeurant compatible avec une résorption rapide du film du matériau collagénique.

Conformément à l'invention, ce compromis est obtenu par la coopération de trois caractéristiques, à savoir :
a) le choix d'un treillis particulier, en l'occurrence un tissu prothétique tridimensionnel, c'est-à-dire ayant une certaine épaisseur séparant ses deux faces ; et une face du tissu est ouverte à toute colonisation cellulaire post-chirurgicale ;
b) une liaison au moins superficielle, voire sur une certaine épaisseur, du film collagénique avec l'autre face dudit tissu, et c'est-à-dire celle opposée à la face ouverte à la colonisation cellulaire ;
c) et le choix d'un matériau collagénique particulier, à savoir un collagène dont la structure hélicoïdale est au moins partiellement thermo-dénaturée sans dégradation hydrolytique.

Cette coopération permet de développer immédiatement la colonisation tissulaire, en toute indépendance de la résorption du film collagénique, qui est elle-même relativement rapide, par exemple de l'ordre d'une dizaine de jours, compte tenu du matériau collagénique retenu, et ce sans compromettre pour autant le caractère monolithe de la prothèse composite.

Grâce à l'invention, les temps d'intégration tissulaire et de résorption du matériau collagénique deviennent du même ordre de grandeur, ce qui veut dire que dans la plupart des cas, au moment où le tissu est complètement intégré et mécaniquement efficace, par exemple sur la face interne de la paroi abdominale, le matériau collagénique se trouve complètement résorbé, ce qui exclut tout pouvoir adhésiogène postérieur de la prothèse composite.

Grâce à l'invention, l'intégration pariétale ou viscérale de la prothèse n'est pas perturbée par la résorption du matériau collagénique.

Par "face ouverte", on entend que ladite face comporte des alvéoles ayant une certaine profondeur selon l'épaisseur du tissu tridimensionnel, ces alvéoles traversant complètement, ou non, l'épaisseur du tissu, d'une face à l'autre. Dans le cas d'une traversée complète des alvéoles, on parlera de tissu prothétique ajouré.

Selon l'invention, préférentiellement la face du film collagénique, opposée au tissu prothétique est sensiblement lisse et non poreuse.

Le tissu prothétique comprend par exemple deux faces poreuses opposées, séparées l'une de l'autre par l'épaisseur dudit tissu, mais reliées l'une à l'autre par des fils de liaison.

A titre d'exemple, l'armure du tissu prothétique détermine dans l'épaisseur de ce dernier, une multiplicité d'alvéoles ou canaux transversaux, sensiblement parallèles les uns aux autres, débouchant de part et d'autre dudit tissu sur les deux faces poreuses respectivement, et dont la section interne est substantiellement exempte de tout fil de liaison.

De préférence, le matériau collagénique comprend du collagène et au moins un additif hydrophile macromoléculaire, chimiquement non réactif vis-à-vis du collagène.

Par l'expression, "chimiquement non réactif vis-à-vis du collagène", on entend un composé qui n'est pas susceptible de réagir avec le collagène présent, notamment qui ne forme pas de liaison covalente avec celui-ci lors de sa réticulation.

Avantageusement, l'épaisseur du film est moins importante que l'épaisseur du tissu prothétique, par exemple comprise entre 2% et 10% de l'épaisseur totale de la prothèse composite, et de préférence comprise entre environ 30 µm et 100 µm, et plus préférentiellement est d'environ 50 µm à 75 µm.

De manière préférée, le matériau collagénique comprend du collagène modifié par coupure oxydative et chauffage au-dessus de 37°C, réticulé en présence d'au moins un additif hydrophile macromoléculaire chimiquement non réactif vis-à-vis dudit collagène.

De manière générale, ce matériau collagénique peut être obtenu par le procédé suivant, qui comprend les étapes consistant à :
- préparer une solution de collagène modifié par coupure oxydative ;
- traiter par chauffage ladite solution à une température supérieure à 37°C ;
- mélanger la solution collagénique résultante avec une solution contenant au moins un additif hydrophile macromoléculaire, chimiquement non réactif vis-à-vis du collagène ;
- ajuster le pH dudit mélange jusqu'à pH neutre.

Le matériau collagénique qui en résulte et qui fait partie de la prothèse composite de l'invention est biocompatible, non toxique, et se résorbe in vivo en moins de deux semaines. Le collagène utilisé pour former la solution collagénique peut être indifféremment d'origine animale ou humaine, mais de préférence est du collagène natif, solubilisé à pH acide ou après traitement par digestion à la pepsine. Il peut s'agir en particulier de collagène bovin de type I ou de collagènes humains de type I ou III, ou encore de mélanges en toutes proportions de ces derniers.

L'étape de coupure oxydative se fait de préférence à l'aide d'acide périodique ou l'un de ses sels, pour le rendre auto-réticulable, et ainsi la réticulation peut être effectuée sans l'aide d'agents réticulants.

Le film est lié au moins superficiellement au tissu prothétique, et de préférence sur une certaine épaisseur par absorption capillaire des fibres constitutives dans le tissu prothétique.

Plus préférentiellement encore, le matériau collagénique se présente sous forme de film lié par absorption capillaire des fibres constitutives du tissu prothétique sur une profondeur inférieure à 750 µm, mesurée à partir la surface extérieure du film.

Avantageusement, l'additif hydrophile macromoléculaire présente un poids moléculaire supérieur à 3000 Daltons, et de préférence est un polymère hydrophile de poids moléculaire compris entre 3000 et 20000 Daltons. De tels additifs sont, par exemple, le polyéthylèneglycol, des polysaccharides, tels que l'amidon, le dextrane, et la cellulose, et des polysaccharides modifiés portant des fonctions carboxyliques. De préférence, l'additif hydrophile macromoléculaire est le polyéthylèneglycol.

La concentration pondérale en additif hydrophile est préférentiellement 2 à 10 fois inférieure à celle du collagène. La réticulation du collagène peut être réalisée à une température comprise entre 4°C et 30°C, et de préférence entre 18°C et 25°C.

Avantageusement, la prothèse composite comporte un film constitué d'un collagène tel que précédemment défini et d'au moins un additif hydrophile macromoléculaire selon un rapport pondéral collagène/additif hydrophile de 1/1 à 9/1, de préférence de 2/1 à 4/1, et plus préférentiellement de 3/1.

Conformément à l'invention, une prothèse composite comporte deux faces différentes dans leurs aspects et fonctions respectifs, à savoir une face poreuse ou ouverte d'un côté, pour accueillir et diriger la colonisation cellulaire post-chirurgicale, et l'autre face fermée, pour la séparation tissulaire sans adhérence.

Le film du matériau collagénique est de préférence continu, lisse et non poreux, recouvrant entièrement le tissu prothétique, et plus préférentiellement, dépasse les bords de ce dernier de façon à protéger la prothèse de contacts viscéraux, le débord pouvant être par exemple de 5 à 10 millimètres.

Le film est intimement lié au tissu par pénétration superficielle, et non délaminable, de façon à ne pas constituer un plan de clivage, tout en maintenant ouverte la porosité sur l'autre face du tissu prothétique.

De préférence, le film est également souple, de façon à préserver la maniabilité du produit et son éventuelle utilisation par voie coelioscopique.

Une fois réhydraté, il restaure au tissu prothétique ses propriétés mécaniques initiales (souplesse et élasticité) sans se fragmenter, ni rendre la fixation de la prothèse plus difficile. Il est en outre transparent, et non collant lors de sa mise en place. Sa résorption rapide assure la protection contre les phénomènes adhésiogènes initiaux, c'est-à-dire dans la première semaine post-opératoire, ou autrement dit, pendant le laps de temps nécessaire à l'intégration de la face opposée. Lors de sa résorption, son caractère faiblement inflammatoire et/ou immunogène ne perturbe pas la colonisation tissulaire à l'opposé dudit film.

La présente invention sera mieux comprise par la description détaillée d'un mode d'exécution préféré, donnée à titre d'exemple, et en se référant aux figures jointes en annexe, dans lesquelles :
- la **Figure 1** est une vue en perspective d'une prothèse composite conformément à l'invention ;
- la **Figure 2** est une image microscopique d'une prothèse conformément à l'invention, prise en perspective du côté du film, à une échelle d'agrandissement de 20 fois la taille réelle ;
- la **Figure 3** est une image microscopique de la prothèse de la Figure 2, à une échelle d'agrandissement de 30 fois la taille réelle ;
- la **Figure 4** est une autre image microscopique de la même prothèse des Figures 2 et 3, à une échelle d'agrandissement de 120 fois la taille réelle ;
- la **Figure 5** est une image microscopique d'une prothèse composite selon l'invention, vue du côté du tissu, à l'échelle d'agrandissement de 25 fois la taille réelle ;
- la **Figure 6** est une image microscopique en coupe transversale d'une prothèse composite selon l'invention, à l'échelle d'agrandissement de 40 fois la taille réelle ;
- la **Figure 7** représente un dessin schématique de l'armure de tricotage d'un tissu prothétique appartenant à une prothèse composite selon la présente invention.

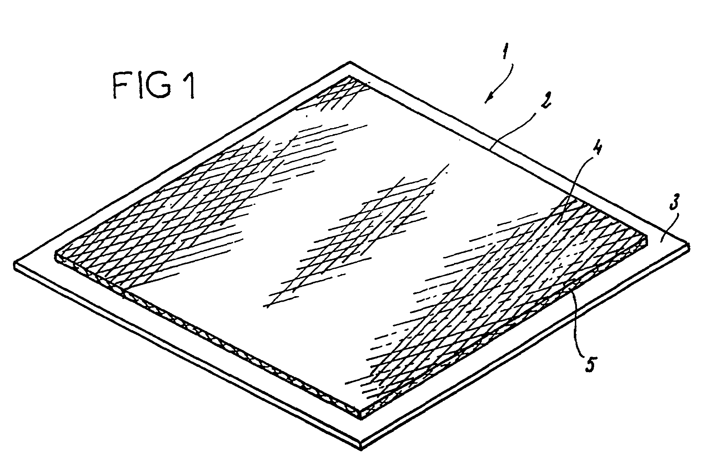

En se référant utilement à la Figure 1, une prothèse composite selon la présente invention est représentée de manière générale par la référence 1. La prothèse comporte un tissu prothétique 2, présentant deux faces 4 et 5, dont l'une est recouverte d'un film de matériau collagénique 3. Le tissu prothétique 2 a une structure tridimensionnelle ajourée, et en particulier en "nid d'abeille", et donc une certaine épaisseur qui sépare la face 4 de la face 5. Ce tissu peut être obtenu de manière préférée avec un tricot Rachel à entretoise réalisé sur double fonture. L'écartement des deux fontures et les débits des fils permettent d'obtenir un tissu fini en trois dimensions (structure tridimensionnelle) d'une épaisseur comprise entre 1 à 3 mm, et par exemple d'environ 1,8 mm, pour un poids d'environ 90 g/m². Les caractéristiques finales du tissu sont données indépendamment du tricotage par le choix de la matière première employée, par exemple du polyester PES 50 dtex multifilaments, la température, et le temps de thermofixage. En dehors de la filature, le fil et le tissu ne reçoivent aucun autre traitement (pas d'ensimage ou de lavage). Un tel tissu sans collagène présente, selon la norme NFG 07119, une force de rupture par traction comprise entre environ 18 daN à environ 30 daN, et un allongement de rupture par traction d'environ 25% à 37%, en chaîne, et une force de rupture par traction comprise entre environ 9 daN à environ 15 daN, et un allongement de rupture par traction de l'ordre de 60% à 88%, en trame.

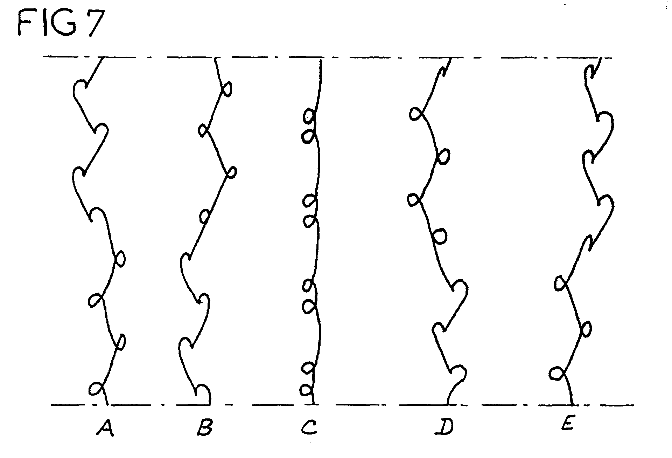

Un tel tissu composite peut être réalisé par tricotage en chaîne de cinq nappes de fils, et conformément au dessin schématique de la Figure 7. Dans cette figure, chaque nappe de fils est identifiée par une lettre, allant de A à E, le schéma en lui-même utilisant un système de description du tricot à réaliser tout à fait habituel et compréhensible pour l'homme du métier, et qui ne sera pas décrit plus en détails ici. Conformément à la 5 Figure 7, le tissu prothétique préféré selon la présente invention est constitué, comme précédemment décrit, de deux faces poreuses indépendantes. Ces deux faces sont, dans l'exemple donné, elles-mêmes constituées de deux nappes de fils, référencées A,B et D,E respectivement, les nappes A,B donnant une face à ouvertures en forme de goutte d'eau, pour accueillir et diriger la colonisation cellulaire post-chirurgicale, et les nappes D,E donnant une face à ouvertures hexagonales qui seront fermées par le film du matériau collagénique. Le tissu prothétique peut être tricoté sur un métier Rachel à double fonture. Dans ce cas, toutes les barres correspondant aux fils A,B et D,E sont enfilées un plein-un vide. La nappe de fils de liaison est représentée par la référence C, et est enfilée pleine. Les différentes nappes A-E de fils sont toutes tricotées en même temps. Ainsi, les fils de liaison sont distribués selon les bords périphériques des ouvertures de chaque face et s'étendent sensiblement perpendiculairement depuis une face vers l'autre face, évitant que des fils de liaison occupent une partie trop importante du volume des canaux transversaux qui sont formés. Le tissu final peut ensuite être stabilisé simplement par passage au four à une température comprise entre environ 170°C et environ 220°C.

La fabrication d'une prothèse composite associant un tissu prothétique à structure ajourée tridimensionnelle, tel que décrit précédemment, avec un film d'un matériau collagénique peut être réalisée comme suit.

La solution contenant le collagène est modifiée préalablement par coupure oxydative, chauffage, avec un additif hydrophile macromoléculaire, et éventuellement de la glycérine. Ensuite, cette solution est répartie uniformément sur un support inerte hydrophobe et plan pour former un film résultant de deux couches minces superposées.

Pour ce faire, on applique une première couche mince de solution dans laquelle les concentrations pondérales en collagène, en additif hydrophile macromoléculaire et en glycérine, si présente, sont de préférence respectivement comprises entre environ 2 et 6%, pour le collagène, environ 0,6 et 2% pour l'additif hydrophile macromoléculaire, environ 0,3 et 0,8% pour la glycérine. Cette première couche mince présente une densité comprise entre environ 0,035 à environ 0,27 g/cm³, ce qui représente environ 70 à 90% de la densité finale en film collagénique présent dans la prothèse composite. Après gélification de cette première couche mince par refroidissement, on applique à sa surface une deuxième couche mince à partir de la même solution, de préférence ajustée à une concentration en éthanol de 5 à 50%. La deuxième couche mince représente environ 10 à 30% de la densité finale en film collagénique présent dans la prothèse composite.

Le tissu prothétique à structure ajourée tridimensionnelle, tel qu'obtenu précédemment, présentant une épaisseur de l'ordre de 1,8 mm, est appliqué par sa face à ouvertures hexagonales sur la deuxième couche mince, avant gélification, de telle sorte que l'ancrage s'effectue pendant le séchage du collagène réticulé. Après réaction, la prothèse composite est séparée du support inerte hydrophobe.

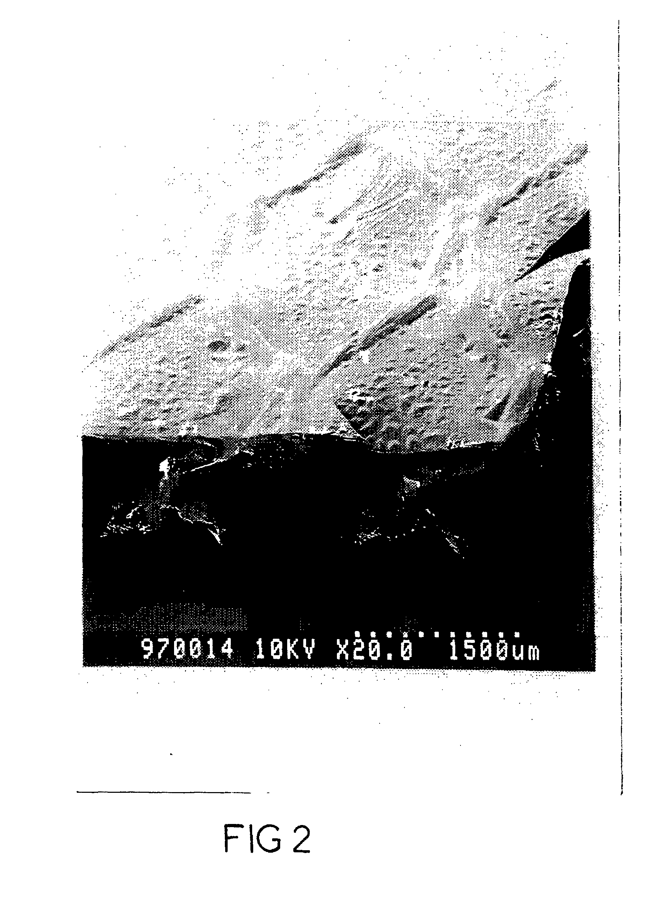

La figure 2 représente une image d'une prothèse composite obtenue de la manière décrite avec un tissu tridimensionnel. Sur la figure 2, on voit très bien l'empreinte de la structure du tissu prothétique dessous le film de matériau collagénique, ainsi que la forme hexagonale des canaux transversaux, et la torsion des fibres constituant le tissu. On aperçoit également en bas de la figure des parties du tissu prothétique en section, qui ressemblent à des "Y" à l'envers.

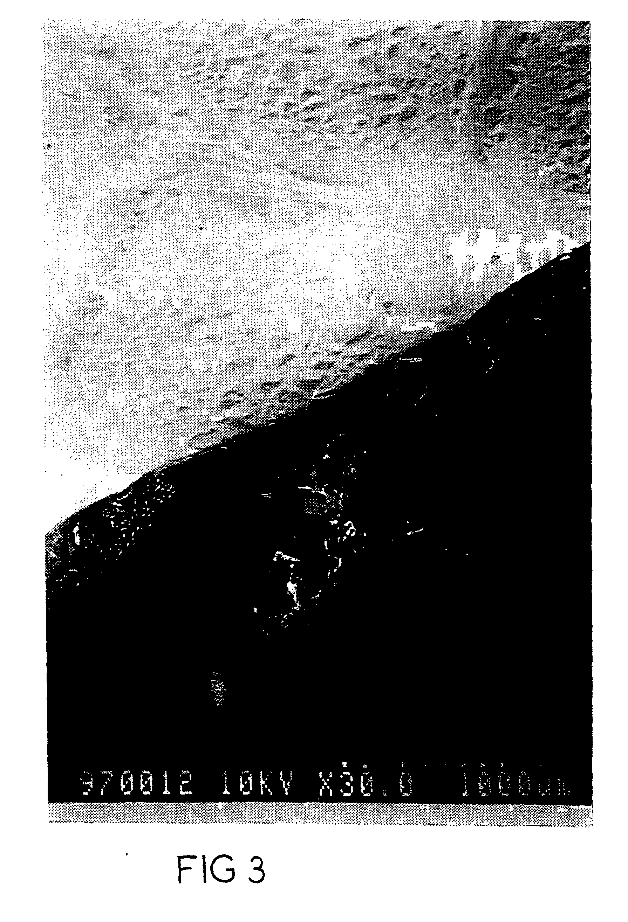

La figure 3 montre la même image, mais à échelle encore plus agrandie (30 fois taille réelle). Cette image montre plus clairement une des parties en "Y" à l'envers du tissu prothétique, ainsi que le bord du film de matériau collagénique.

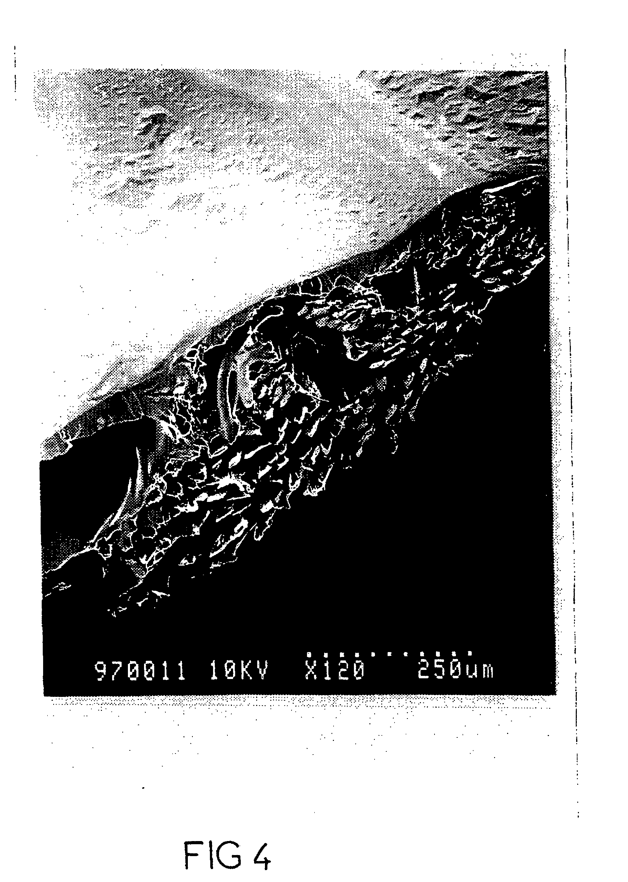

La figure 4 montre la même prothèse à l'échelle agrandie à 120 fois la taille réelle. On aperçoit clairement sur cette image le bord du film de matériau collagénique qui, lors de la section pour les besoins de la photographie, s'étend au-delà du tissu prothétique sous la forme d'une jupe de matériau. Sur la partie gauche inférieure, près du bord gauche de l'image, on remarque également que le matériau collagénique est remonté par capillarité dans les fibres, cet effet étant en partie responsable de la résistance à la délamination élevée du film du tissu prothétique. Enfin, on remarque que sur l'ensemble des Figures 2 à 4, le film est continu et lisse, aucune fibre synthétique n'apparaissant à la surface.

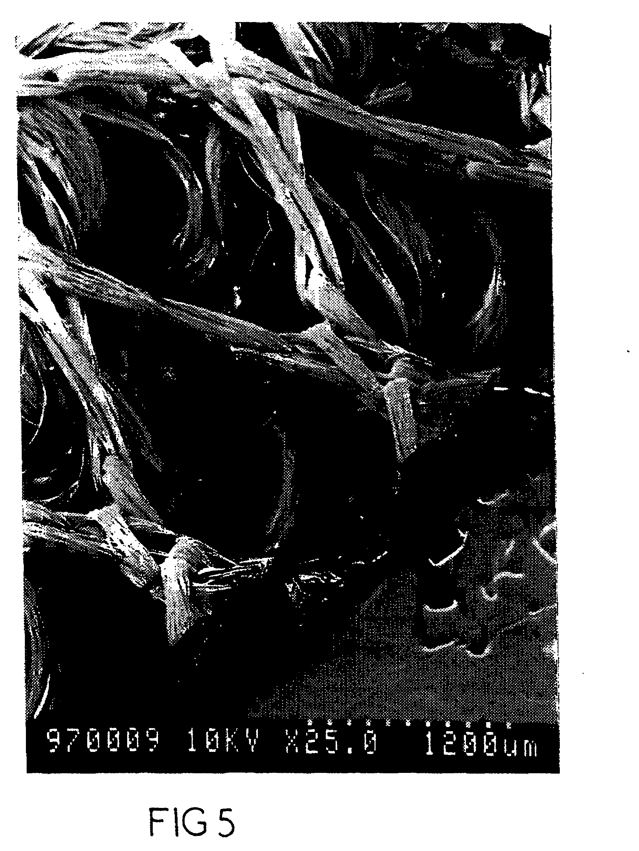

La figure 5 montre une image à l'envers de la même prothèse, c'est-à-dire vue du côté du tissu, et illustre clairement la structure ajourée tridimensionnelle du tissu prothétique, avec en-dessus le film collagénique qui présente une face interne complètement lisse.

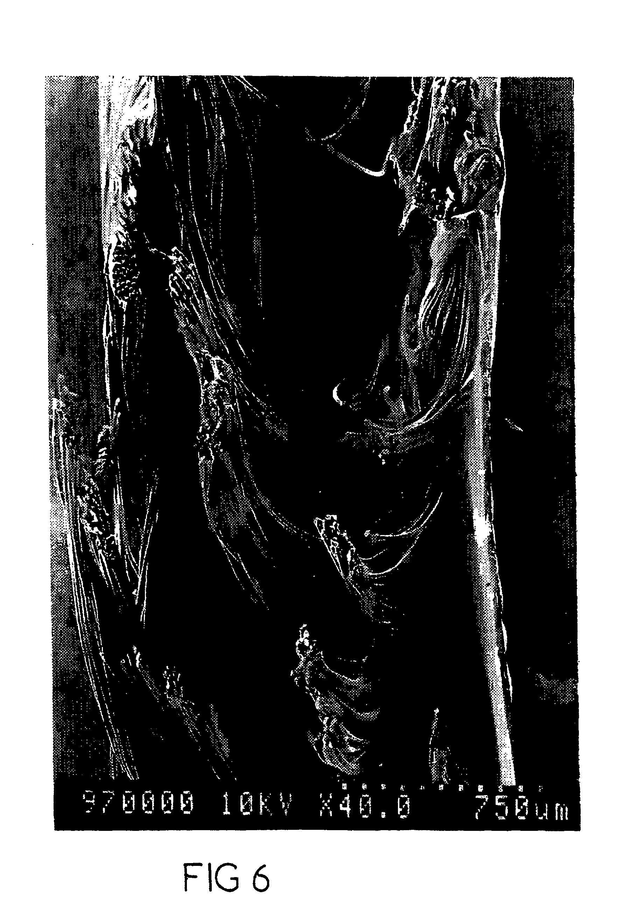

Enfin, la figure 6 montre une image en coupe transversale, à l'échelle de 40 fois la taille réelle de la même prothèse, pour donner une idée de l'épaisseur du film de matériau collagénique. Celui-ci se trouve légèrement à droite d'une ligne médiane imaginaire, le bloc foncé totalement à droite de l'image étant le support de photographie. Ainsi, on aperçoit que le film présente une épaisseur de l'ordre de 50 µm à 75µm, mais que la matériau collagénique a également été absorbé par capillarité dans les fibres du tissu prothétique jusqu'à une épaisseur d'environ 750 µm.

Des essais cliniques ont été menés sur un modèle d'abrasion caecale chez le rat, selon le protocole décrit par Harris et Coll. (1995), dans "Analysis of the Kinetics of Peritoneal Adhesion Formation in the Rat and Evaluation of Potential Antiadhesive Agents", Surgery, 117(6), pages 663-669, qui est largement utilisé et validé pour les études des adhérences post-chirurgicales. Les essais consistent à créer une abrasion et une déshydratation sur des surfaces de 2 cm² de paroi péritonéale et de caecum, au contact l'une de l'autre. Le groupe témoin ne reçoit aucun produit de protection des plaies ainsi créées. Il est comparé aux groupes de rats qui reçoivent un produit de protection, conformément au Tableau 1 ci-dessous :

**TABLEAU I**

| | Incidence des adhésions surface à surface (nombre d'animaux / nombre d'animaux totaux) | Surface d'adhésion (cm²) | Pourcentage de surface de l'adhésion par rapport à la surface de la lésion | Force maximum pour rompre l'adhésion formée (N) |
|---|---|---|---|---|
| Témoin | 44/45 | 1,27 | 63 | 1,25 |
| Interposition d'un film de matériau collagénique | 0/10 | 0 | 0 | 0 |
| Interposition d'un tissu prothétique ajouré tridimensionnel | 12/12 | 1,62 | 81 | 1,28 |
| Interposition d'une prothèse composite selon l'exemple de l'invention | 1/12 | 0,11* | 6* | 0,37* |
| Interposition d'un film à base d'acide hyaluronique (SEPRAFILM® ) | 2/10 | 0,85 | 43 | 0,93 |

| | | | | |
|---|---|---|---|---|
| * = valeurs obtenues pour l'unique animal ayant développé une adhérence caeco-pariétale. | | | | |

Les essais effectués avec une prothèse composite selon l'invention montrent qu'elle permet d'éviter des adhérences dès le début de son implantation dans le corps, et que le film en particulier est résorbé in vivo en moins d'une semaine. La colonisation du tissu prothétique s'effectue par l'autre face dans l'épaisseur de la structure tridimensionnelle, de telle sorte que celui-ci est totalement intégré dans le corps pour remplir complètement sa fonction de renfort, notamment dans le traitement des éventrations, lorsque le film se trouve résorbé.

Ces résultats ont été confirmés 6 semaines post-opératoires, l'absence totale d'adhérences ayant été constatée.

## Revendications

1. Prothèse composite, comprenant un tissu prothétique, et au moins un film d'un matériau collagénique réticulé, associé à une face du tissu prothétique, **caractérisée en ce que** le tissu prothétique a une structure tridimensionnelle séparant ses deux faces, dont au moins l'une est ouverte à toute colonisation cellulaire post-chirurgicale, et le film de matériau collagénique est lié au moins superficiellement à l'autre face dudit tissu, et ledit matériau collagénique comporte du collagène dont la structure hélicoïdale est au moins partiellement thermo-dénaturée sans dégradation hydrolytique.

2. Prothèse selon la revendication 1, **caractérisée en ce que** le tissu prothétique a une structure ajourée.

3. Prothèse selon la revendication 1, **caractérisée en ce que** le matériau collagénique comprend au moins un additif hydrophile macromoléculaire, chimiquement non réactif vis-à-vis du collagène.

4. Prothèse composite selon la revendication 1, **caractérisée en ce que** l'épaisseur du film est moins importante que l'épaisseur du tissu prothétique.

5. Prothèse composite selon la revendication 1, **caractérisée en ce que** l'épaisseur du film est comprise entre 2% et 10% de l'épaisseur totale de la prothèse composite.

6. Prothèse composite selon la revendication 1, **caractérisée en ce que** l'épaisseur du film est comprise entre environ 30 µm à 100 µm, et de préférence est comprise entre environ 50°µm à 75 µm.

7. Prothèse composite selon la revendication 1, **caractérisée en ce que** le matériau collagénique comprend du collagène modifié par coupure oxydative et chauffage au-dessus de 37°C, réticulé en présence d'au moins un additif hydrophile macromoléculaire chimiquement non réactif vis-à-vis dudit collagène.

8. Prothèse composite selon la revendication 7, **caractérisée en ce que** le collagène est modifié par coupure oxydative à l'aide d'acide périodique ou l'un de ses sels pour le rendre auto-réticulable.

9. Prothèse selon la revendication 1, **caractérisée en ce que** le film est lié par absorption capillaire aux fibres constitutives du tissu prothétique.

10. Prothèse selon la revendication 9, **caractérisée en ce que** le film est lié au tissu prothétique sur une profondeur inférieure à 750 µm, mesurée à partir de la surface extérieure du film.

11. Prothèse selon la revendication 3, **caractérisée en ce que** l'additif hydrophile macromoléculaire a un poids moléculaire supérieur à 3000 Daltons.

12. Prothèse selon la revendication 11, **caractérisée en ce que** l'additif hydrophile macromoléculaire est un polymère hydrophile de poids moléculaire compris entre 3000 et 20000 Daltons.

13. Prothèse selon la revendication 12, **caractérisée en ce que** l'additif hydrophile macromoléculaire est le polyéthylèneglycol.

14. Prothèse selon la revendication 3, **caractérisée en ce que** le matériau collagénique présente un rapport collagène/additif hydrophile compris entre 1:1 à 9:1, de préférence de 2:1 à 4:1, et plus préférentiellement de 3:1.

15. Prothèse selon la revendication 1, **caractérisée en ce que** le tissu prothétique comprend deux faces poreuses opposées, reliées l'une à l'autre par des fils de liaison, dont l'une est ouverte à toute colonisation cellulaire post-chirurgicale, et dont l'autre est fermée à ladite colonisation par le film matériau collagénique.

## Patentansprüche

1. Verbundprothese, mit einem Prothesengewebe und zumindest einem Film aus einem vernetzten Kollagenmaterial, der mit einer Seite des Prothesengewebes verbunden ist, **dadurch gekennzeichnet, daß** das Prothesengewebe eine dreidimensionale Struktur aufweist, die seine beiden Seiten trennt, von der zumindest die eine für jegliche post-chirurgische zelluläre Kolonisierung zugänglich ist, daß der Film aus dem Kollagenmaterial zumindest oberflächlich an die andere Seite des Gewebes gebunden ist, und daß das Kollagenmaterial Kollagen aufweist, dessen Helix-Struktur zumindest teilweise ohne hydrolytische Degradation thermo-denaturiert ist.

2. Verbundprothese nach Anspruch 1, **dadurch gekennzeichnet, daß** das Prothesengewebe eine durchbrochene Struktur aufweist.

3. Verbundprothese nach Anspruch 1, **dadurch gekennzeichnet, daß** das Kollagenmaterial zumindest ein hydrophiles makromolekulares Additiv aufweist, das gegenüber Kollagen chemisch nicht reaktiv ist.

4. Verbundprothese nach Anspruch 1, **dadurch gekennzeichnet, daß** die Dicke des Films geringer ist als die Dicke des Prothesengewebes.

5. Verbundprothese nach Anspruch 1, **dadurch gekennzeichnet, daß** die Dicke des Films im Bereich von 2 % bis 10 % der Gesamtdicke der Verbundprothese liegt.

6. Verbundprothese nach Anspruch 1, **dadurch gekennzeichnet, daß** die Dicke des Films im Bereich von etwa 30 µm bis 100 µm, und vorzugsweise im Bereich von etwa 50 µm bis 75 µm, liegt.

7. Verbundprothese nach Anspruch 1, **dadurch gekennzeichnet, daß** das Kollagenmaterial durch oxydative Freisetzung und Erwärmung oberhalb von 37 °C modifiziertes Kollagen aufweist, das in Anwesenheit zumindest eines hydrophilen makromolekularen Additivs vernetzt worden ist, das gegenüber Kollagen chemisch nicht reaktiv ist.

8. Verbundprothese nach Anspruch 7, **dadurch gekennzeichnet, daß** das Kollagen durch oxydative Freisetzung mittels Periodsäure oder einem ihrer Salze modifiziert ist, um es selbst-vernetzbar zu machen.

9. Verbundprothese nach Anspruch 1, **dadurch gekennzeichnet, daß** der Film durch kapillare Absorption an die das Prothesengewebe bildenden Fasern gebunden ist.

10. Verbundprothese nach Anspruch 9, **dadurch gekennzeichnet, daß** der Film an das Prothesengewebe über eine Tiefe von weniger als 750 µm, gemessen von der äußeren Oberfläche des Films, gebunden ist.

11. Verbundprothese nach Anspruch 3, **dadurch gekennzeichnet, daß** das hydrophile makromolekulare Additiv ein Molekulargewicht von mehr als 3.000 Daltons aufweist.

12. Verbundprothese nach Anspruch 11, **dadurch gekennzeichnet, daß** das hydrophile makromolekulare Additiv ein hydrophiles Polymer mit einem Molekulargewicht im Bereich von 3.000 bis 20.000 Daltons ist.

13. Verbundprothese nach Anspruch 12, **dadurch gekennzeichnet, daß** das hydrophile makromolekulare Additiv Polyethylenglykol ist.

14. Verbundprothese nach Anspruch 3, **dadurch gekennzeichnet, daß** das Kollagenmaterial ein Verhältnis Kollagen/hydrophiles Additiv im Bereich von 1:1 bis 9:1, vorzugsweise von 2:1 bis 4:1, und besonders bevorzugt von 3:1, aufweist.

15. Verbundprothese nach Anspruch 1, **dadurch gekennzeichnet, daß** das Prothesengewebe zwei miteinander durch Verbindungsfäden verbundene poröse Seiten aufweist, von denen die eine für jegliche post-chirurgische zelluläre Kolonisierung zugänglich ist, und von denen die andere durch den Film aus Kollagenmaterial für diese Kolonisierung unzugänglich ist.

## Claims

1. Composite prosthesis, comprising a prosthetic fabric and at least one film of a crosslinked collagenous material associated with one surface of the prosthetic fabric, **characterized in that** the prosthetic fabric has a three-dimensional structure separating its two surfaces, at least one of which is open to any postsurgical cell colonization, and the film of collagenous material is linked, at least on the surface, to the other surface of said fabric, and said collagenous material comprises collagen whose helical structure is, at least partially, thermally denatured without hydrolytic degradation.

2. Prosthesis according to claim 1, **characterized in that** the prosthetic fabric has an openwork structure.

3. Prosthesis according to claim 1, **characterized in that** the collagenous material comprises at least one macromolecular hydrophilic additive chemically unreactive with respect to collagen.

4. Composite prosthesis according to claim 1, **characterized in that** the thickness of the film is less than the thickness of the prosthetic fabric.

5. Composite prosthesis according to claim 1, **characterized in that** the thickness of the film is between 2% and 10% of the total thickness of the composite prosthesis.

6. Composite prosthesis according to claim 1, **characterized in that** the thickness of the film is between approximately 30 µm and 100 µm and preferably is between about 50µm and 75 µm.

7. Composite prosthesis according to claim 1, **characterized in that** the collagenous material comprises collagen modified by oxidative scission and heating above 37°C, crosslinked in the presence of at least one macromolecular hydrophilic additive chemically unreactive with respect to said collagen.

8. Composite prosthesis according to claim 7, **characterized in that** the collagen is modified by oxidative scission with the aid of periodic acid or one of its salts in order to make it self-crosslinkable.

9. Prosthesis according to claim 1, **characterized in that** the film is linked by capillary absorption to the constituent fibers of the prosthetic fabric.

10. Prosthesis according to claim 9, **characterized in that** the film is linked to the prosthetic fabric over a depth of less than 750 µm, measured from the outer surface of the film.

11. Prosthesis according to claim 3, **characterized in that** the macromolecular hydrophilic additive has a molecular weight of greater than 3000 daltons.

12. Prosthesis according to claim 11, **characterized in that** the macromolecular hydrophilic additive is a hydrophilic polymer having a molecular weight of between 3000 and 20,000 daltons.

13. Prosthesis according to claim 12, **characterized in that** the macromolecular hydrophilic additive is polyethylene glycol.

14. Prosthesis according to claim 3, **characterized in that** the collagenous material has a collagen/hydrophilic additive ratio of between 1:1 and 9:1, preferably from 2:1 to 4:1 and more preferably 3:1.

15. Prosthesis according to claim 1, **characterized in that** the prosthetic fabric comprises two opposed porous surfaces, connected to each other by linking yarns, one of which is open to any postsurgical cell colonization and the other of which is closed to said colonization by the film of collagenous material.
